# EUROPÄISCHE PATENTSCHRIFT

(11) **EP 1 572 027 B1**
(45) Veröffentlichungstag und Bekanntmachung des Hinweises auf die Patenterteilung: **09.02.2011**
(21) Anmeldenummer: 03767777.0
(22) Anmeldetag: 10.12.2003
(51) Int. Cl.: A61F 2/02, A61L 27/00

(54) **Implantate beschichtet mit Aptameren, die die Adhäsion von endothelialen Vorläuferzellen vermitteln**
Implants coated with Aptamere which mediate the adhesion of endothelial progenitor cells
Implants revêtés d'aptamères qui causent une adhésion de cellules endothéliales progénitrices

(30) Priorität: 17.12.2002 DE 10258924
(43) Veröffentlichungstag der Anmeldung: 14.09.2005
(73) Patentinhaber: Eberhard-Karls-Universität Tübingen, 72076 Tübingen (DE)
(72) Erfinder: SCHLÜSENER, Hermann, 72076 Tübingen (DE); WENDEL, Hans-Peter, 72336 Balingen (DE)
(74) Vertreter: Findeisen, Marco
(86) Internationale Anmeldenummer: PCT/EP2003/013989
(87) Internationale Veröffentlichungsnummer: WO 2004/055153

(56) Entgegenhaltungen:
- WO-A-01/92566
- DE-A- 10 019 154
- DE-A- 19 745 668
- DE-A- 19 755 801
- YODER M C: "Defining human endothelial progenitor cells." JOURNAL OF THROMBOSIS AND HAEMOSTASIS : JTH JUL 2009 LNKD- PUBMED:19630767, vol. 7 Suppl 1, July 2009 (2009-07), pages 49-52, ISSN: 1538-7836

### SEQUENZPROTOKOLL

<110> Eberhard-Karls-Universität Tübbingen
<120> Mit die Adhäsion von biologischem Material vermittelnden Substanzen beschichtete Vorrichtungen
<130> 5402P222W0
<160> 17
<170> PatentIn version 3.1
<210> 1
   <211> 23
   <212> DNA
   <213> Künstliche Sequenz
<220>
   <223> Nukleotidsequenz
<400> 1
   tcgtcgtttt gtcgttttgt cgt 23
<210> 2
   <211> 21
   <212> DNA
   <213> Künstliche Sequenz
<220>
   <223> Nukleotidsequenz
<400> 2
   ccgccgccgc cgccgccgcc g 21
<210> 3
   <211> 21
   <212> DNA
   <213> Künstliche Sequenz
<220>
   <223> Nukleotidsequenz
<400> 3
   gcggcggcgg cggcggcggc g 21
<210> 4
   <211> 21
   <212> DNA
   <213> Künstliche Sequenz
<220>
   <223> Nukleotidsequenz
<400> 4
   cggcggegge ggcggcggcg g 21
<210> 5
   <211> 21
   <212> DNA
   <213> Künstliche Sequenz
<220>
   <223> Nukleotidsequenz
<400> 5
   ttcttcttct tcttcttctt c 21
<210> 6
   <211> 25
   <212> DNA
   <213> Künstliche Sequenz
<220>
   <223> Nukleotidsequenz
<400> 6
   ttaggttagg ttaggttagg ttagg 25
<210> 7
   <211> 15
   <212> DNA
   <213> Künstliche Sequenz
<220>
   <223> Nukleotidsequenz
<400> 7
   gctagacgtt agcgt 15
<210> 8
   <211> 15
   <212> DNA
   <213> Künstliche Sequenz
<220>
   <223> Nukleotidsequenz
<400> 8
   gctagagctt aggct 15
<210> 9
   <211> 20
   <212> DNA
   <213> Künstliche Sequenz
<220>
   <223> Nukleotidsequenz
<400> 9
   gattgcctga cgtcagagag 20
<210> 10
   <211> 20
   <212> DNA
   <213> Künstliche Sequenz
<220>
   <223> Nukleotidsequenz
<400> 10
   ttcatgacgt tcctgatcgt 20
<210> 11
   <211> 20
   <212> DNA
   <213> Künstliche Sequenz
<220>
   <223> Nukleotidsequenz
<400> 11
   tccatgactt tcctcaggtt 20
<210> 12
   <211> 20
   <212> DNA
   <213> Künstliche Sequenz
<220>
   <223> Nukleotidsequenz
<400> 12
   tccatgagct tcctgatgct 20
<210> 13
   <211> 24
   <212> DNA
   <213> Künstlich Sequenz
<220>
   <223> Nukleotidsequenz
<400> 13
   tgctgctttt gtgcttttgt gctt 24
<210> 14
   <211> 34
   <212> DNA
   <213> Künstliche Sequenz
<220>
   <223> Nukleotidsequenz
<400> 14
   ccgcggccag gctagctaca acgacctgga cgat 34
<210> 15
   <211> 59
   <212> DNA
   <213> Künstliche Sequenz
<220>
   <223> Nukleotidsequenz
<400> 15
   attgtggttg gtagtataca tttttccgcg gccaggctag ctacaacgac ctggacgat 59
<210> 16
   <211> 34
   <212> DNA
   <213> Künstliche Sequenz
<220>
   <223> Nukleotidsequenz
<400> 16
   ctttaatgcg gggtaatttc ttttccataa tcgc 34
<210> 17 <211> 40
   <212> DNA
   <213> Künstliche Sequenz
<220>
   <223> Nukleotidsequenz
<400> 17
   ttatttccct tctgtatata gatatgctaa atccttactt 40

## Beschreibung

Die vorliegende Erfindung betrifft Implantate mit zumindest einer mit Geweben und/oder Flüssigkeiten des menschlichen oder tierischen Körpers in Kontakt kommenden Oberfläche, welche zumindest teilweise mit beschichtet ist, die die Adhäsion von endothelialen Vorläuferzellen vermitteln, dadurch gekennzeichnet, dass die Substanzen Aptamere sind.

Vorrichtungen mit beschichteten Oberflächen spielen insbesondere dann eine große Rolle, wenn solche Vorrichtungen in Kontakt mit humanem Gewebe oder Blut kommen, wie es bspw. bei einem extrakorporalen Blutkreislauf-System oder bei Gefäßprothesen der Fall ist.

Bei einem extrakorporalen Blutkreislauf, der bspw. bei Operationen am offenen Herzen oder bei der Dialyse eingesetzt werden muß, kommt Blut mit künstlichen Oberflächen, z.B, von Schläuchen, Pumpen, Oxygenatoren etc., zumindest kurzzeitig in Kontakt. Durch diesen Kontakt können im Blut Gerinnungs- und Verklumpungsreaktionen ausgelöst werden, durch welche u.a. lebensgefährliche Thrombosen, Entzündungsreaktionen und Biofilmbildung (bakterielle Besiedlung) hervorgerufen werden können. Bei Herz- und/oder Lungenunterstützungssystemen, außerdem bei Katheter und Gefäßzugängen kann sogar ein Kontakt über mehrere Wochen hinweg vorgesehen sein. Auch Aufbewahrungssysteme für Blutkomponenten oder bspw. Kontaktlinsen können über einen längeren Zeitraum in Kontakt mit menschlichem oder tierischem Gewebe kommen.

Ferner kommen als mit menschlichem Blut oder Gewebe in Kontakt kommende Vorrichtungen Implantate in Frage, die für eine bestimmte Zeit oder auf Dauer in den menschlichen Körper eingesetzt werden. Dauerhaft eingesetzt werden z.B. künstliche Herzklappen, künstliche Hüft- oder Kniegelenke, Herzschrittmacher und Zahnimplantate, vorübergehend bspw. Platten und Schrauben aus künstlichem (Metall, Keramik, Kunststoff) oder tierischem, immunologisch möglichst inertem Material. Ferner kommen Gefäßprothesen, Conduits, Patches, Katheter, künstliche Blasen etc. in Frage, die prinzipiell aus allen Polymerkunststoffen, Metallen, Legierungen, Textilien, Naturstoffen (Chitosan, Bakterienzellulose, etc.) oder auch aus anderen degradierbaren Materialien bestehen können.

Weiterhin werden in der Gefäßchirurgie häufig Prothesen bspw. in Form von Stents eingesetzt, wobei diese aus verschiedenen Kunststoffen oder Metallen angefertigt werden. Da es sich hierbei um körperfremde Strukturen handelt, sind Entzündungsreaktionen, die Einkapselung der fremden Struktur durch Proliferation des umgebenden Gewebes als Abstoßungsreaktion, sowie Komplikationen in der Blutgerinnung und Restenosen immer wieder zu beobachten.

Nicht nur bei den genannten Anwendungen besteht die Notwendigkeit, die Oberflächen derart zu beschichten, dass sie gegenüber Blut oder anderen Gewebeteilen eine gute Biokompatibilität aufweisen. Biokompatibilität bedeutet im weitesten Sinne die Verträglichkeit von Substanzen mit lebendem biologischen Material (Knochen, Gewebe, Blut, Organe, etc.). Um Komplikationen wie die Koagulation, Proliferation, inflammatorische Reaktionen und Abstoßungsreaktionen zu umgehen, werden heutzutage Vorrichtungen, die in Kontakt mit Blut, Gewebe, etc. kommen, mit biokompatiblen Materialien beschichtet. Die heute eingesetzten Materialien sollen sich im Körper inert verhalten und den Stoffwechsel nicht merklich beeinflussen.

Im Stand der Technik ist bspw. der Ansatz bekannt, Implantate *in vitro* mit patienteneigenen Zellen zu besiedeln. Im Rahmen dieses Konzeptes wird autologes Gewebe *in vitro* aufgearbeitet und kultiviert, und in Verbindung mit geeigneten Matrizes ein an den Patienten "angepasstes" Implantat erzeugt. Dieses Implantat wird anschließend in den Körper des Spenders eingebracht und reift im Empfänger zu einem möglichst naturalistischen Gewebe heran. Dadurch soll verhindert werden, dass Implantate vom Körper als fremd erkannt und abgestoßen werden.

Nachteilig an der Herstellung von Implantaten, die mittels "Tissue engineering" *in vitro* mit Zellen besiedelt werden, ist, dass die Besiedelung solcher Implantate oder Vorrichtungen äußerst aufwendig und unter strengsten sterilen Bedingungen durchgeführt werden muss, um ausreichend gute Erfolge erzielen zu können. Für solche implantierbaren Vorrichtungen müssen zunächst Zellen des Patienten isoliert werden, bei dem ein derartiges Implantat angewendet werden soll. Anschließend müssen die Zellen auf dem betreffenden Implantat kultiviert und vermehrt werden, und schließlich muss das Implantat in den Körper des Patienten operativ eingebracht werden. All diese Schritte machen dieses Verfahren äußerst zeitaufwendig und kostspielig.

Ferner stellte man darüber hinaus häufig fest, dass sich die Zellen auf der Oberfläche der Implantate zwar vermehrt haben, dass sie aber gleichzeitig viele Eigenschaften durch Dedifferenzierung verloren haben. So bilden isolierte Knorpelzellen z.B. kaum oder nur noch eine atypische Knorpelsubstanz. Darmepithel- oder Nierentubuluszellen können nicht mehr in der bekannten Weise Stoffe aufnehmen und haben wesentlich geringere Transport- und Abdichtungsfunktionen.

Im Stand der Technik ist es ferner bekannt, über zellbindende Peptide oder- Proteine die Adhäsion von Zellen an eine mit diesen Peptiden/Proteinen beschichtete Oberfläche zu vermitteln.

In der wissenschaftlichen Literatur wird insbesondere die Verwendung von integrinspezifischen Peptiden oder von Laminin-Abkömmlingen für die Beschichtung von Implantaten beschrieben (siehe bspw. Kantlehner et al., "Surface coating with cyclic RGD peptides stimulates osteoblast adhesion and proliferation as well as bone formation", Chembiochem 18:107-114, 2000; Tamura et al., "Coating of titanium alloy with soluble laminin-5 promotes cell attachment and hemidesmosome assembly in gingival epithelial cells: potential application to dental implants", J. Periodontal Res. 32(3): 287-294, 1997; Kaushal S. et al., "Functional small-diameter neovessels created using endothelial progenitor cells expanded t", Nat. Med. 7(9): 1035-1040, 2001.).

Die DE 197 55 801 offenbart ferner Implantate, die zur zielgerichteten Adhäsionsstimulierung von Körperzellen Peptide enthalten, welche Sequenzen aufweisen, die Bindungsstellen auf den Integrin-Rezeptoren von Zellen erkennen. Diese Peptide sind dabei in einem bestimmten Muster auf der Oberfläche des Implantats angeordnet.

Die Verwendung von Peptiden hat jedoch den Nachteil, dass diese in Konstruktion und Synthese sehr aufwendig sind, was somit auch die Herstellung von mit diesen Peptiden beschichteten Vorrichtungen aufwendig und teuer macht.

Vor diesem Hintergrund ist es Aufgabe der vorliegenden Erfindung, Implantate mit einer die Adhäsion von biologischem Material vermittelnden Substanz bereitzustellen, die kostengünstig und ohne großen Zeitaufwand hergestellt werden können, wobei die Vorrichtungen gleichzeitig gute biokompatible Eigenschaften aufweisen und einfach mit Zellen und/oder Proteinen besiedelt werden können.

Erfindungsgemäß wird die Aufgabe durch die Bereitstellung eines Implantats gemäß Anspruch 1 gelöst.

Die Erfinder haben erkannt, dass es möglich ist, Oberflächen mit Aptameren derart zu beschichten, dass über diese Aptamere biologisches Material an den Oberflächen immobilisiert werden kann.

Dies war auch deshalb überraschend, da im Stand der Technik die Verwendung von Aptameren in einem anderen Zusammenhang beschrieben werden. So offenbart bspw. die DE 197 45 668 die Verwendung von Aptameren zur Beschichtung eines diagnostischen Chips, d.h. zur extrakorporalen Verwendung. In der WO 01/92566 wird eine Aptamer-beschichtete Vorrichtung zur extrakorporalen Blutreinigung beschrieben.

Aptamere sind hochaffine RNA- oder DNA-Oligo- bzw. Polynukleotide, die aufgrund ihrer spezifischen räumlichen Struktur eine hohe Affinität zu einem Zielmolekül besitzen.

Aptamere sind oft sogar noch spezifischer als Antikörper, und weisen vergleichbare Antigen-Bindungseigenschaften wie Antikörperfragmente auf. Mit ihrer relativ großen und flexiblen Oberfläche können sie potentiell mit mehr Zielmolekülen interagieren als kleinere Moleküle.

Mittels "SELEX" (Systematic evolution of ligands by exponential enrichment) können große Mengen an Oligonukleotiden unterschiedlichster Sequenzen und Sekundärstrukturen enzymatisch erzeugt werden. Aus diesem Pool werden anschließend Oligonukleotide mit hoher Affinität zu einem Zielmolekül herausgesucht und angereichert. Bei Kenntnis der Primärstruktur eines solchen Oligonukleotids kann dieses auch chemisch synthetisiert werden. Ein beispielhaftes Verfahren zur Gewinnung von geeigneten Aptameren ist bspw. beschrieben in der DE 100 19 154.

Die gefundenen Aptamere können dann ferner mittels geeigneter Techniken modifiziert werden, so dass sie geschützt sind und im biologischen Milieu ihre Wirksamkeit nicht verlieren, bspw. nicht von Nukleasen verdaut werden. Schutzmechanismen, die für diesen Zweck geeignet sind, sind im Stand der Technik hinreichend bekannt und schließen bspw. LNA(locked nucleic acids)-Technologien mit Furanose mit ein (siehe bspw.: Wahlestedt et al., "Patent and nontoxic antisense oligonucleotides containing locked nucleic acids", Proc. Natl. Acad. Sci., USA 97(10): 5633-5638, 2000), oder die Spiegelmer®-Technologie der Firma Noxxon (Berlin Deutschland)).

Erfindungsgemäß können jetzt Implantate bereitgestellt werden, deren Oberflächen zumindest teilweise mit bestimmten Aptameren beschichtet sind, über die natives endotheliale Vorläuferzellen an der Oberfläche immobilisiert werden kann.

Vorteilhaft an einer derartigen Aptamerbeschichtung ist es, dass diese Beschichtung stabil und sterilisierbar ist, wodurch Implantate, die mit Aptameren beschichtet werden, kostengünstig hergestellt werden können. Verglichen mit Peptiden besteht ein weiterer Vorteil darin, dass Peptide oft durch die Sterilisation ihre Aktivität verlieren, während Oligonukleotide, also Aptamere, äußerst stabil sind.

Dabei ist es nicht immer notwendig, alle Oberflächen der Implantate zu beschichten, sondern vielmehr z.T. auch wünschenswert, nur bestimmte Flächen auf dem Implantat mit den Aptameren oder unterschiedliche Flächen eines Implantates mit verschiedenen Aptameren zu beschichten. Auf diese Weise kann erreicht werden, dass endotheliale Vorläuferzellen auch nur an die mit den Aptameren beschichtete Oberflächen bindet.

Dies ist bspw. bei Stents, Gefäßprothesen, -zugängen, Ports oder Conduits von Vorteil, welche auf der inneren Oberfläche, die bspw. mit Blut in Kontakt kommt, anders beschichtet werden können als deren äußere Oberfläche, die mit dem den Stent umgebenden Gewebe in Kontakt kommt und in dieses einwachsen soll.

Bei den erfindungsgemäß beschichteten Implantaten erfolgt eine Fixierung von endothelialen Vorläuferzellen aus Blut, Gewebe, Organen oder anderen Quellen, wodurch eine Generierung von autologen funktionellen Grenzfläche, Schichten, Zellverbänden erzielt wird, die somit vom Körper nicht mehr als fremd erkannt werden und die funktionellen physiologischen Eigenschaften des jeweiligen Einsatzortes oder Organs übernehmen.

In einer bevorzugten Ausführungsform sind die Aptamere Nukleinsäuremoleküle, die zumindest eine der Sequenzen SEQ ID-Nr. 1 bis SEQ ID-Nr. 17 aus dem beiliegenden Sequenzprotokoll enthalten.

Die Erfinder konnten in eigenen Versuchen zeigen, dass Nukleinsäuremoleküle, die zumindest eine der vorstehenden Nukleotidsequenzen enthalten, native endotheliale Vorläuferzellen zen enthalten, zeichnen sich nach Erkenntnis der Erfinder durch eine hohe Spezifität für die endothelialen Vorläuferzellen aus.

Bevorzugt ist es ferner, wenn das Nukleinsäuremolekül ein Nukleinsäuremolekül mit einer der Nukleotidsequenzen SEQ ID-Nr. 1 bis SEQ ID-Nr. 17 aus dem beiliegenden Sequenzprotokoll ist.

Nukleinsäuremoleküle mit den offenbarten Sequenzen erwiesen sich als besonders geeignet, endotheliale Vorläuferzellen zu binden.

Dabei ist bevorzugt, wenn die Aptamere entweder direkt und/oder über ein Linkermolekül auf der Oberfläche des Implantates angebracht sind.

Unter "Linkermolekül" oder "Linker" ist hierbei jede Substanz zu verstehen, mit der ein Aptamer auf der Oberfläche angebracht werden kann.

Dabei ist bevorzugt, wenn das Linkermolekül N-Succinimidyl 3-(2-pyridyldithio)propionat oder/und ein bspw. lineares oder sternförmiges PEG-Block-Copolymer ist.

Von der Substanz N-Succinimidyl 3-(2-pyridyldithio)propionat konnte gezeigt werden, dass sie bereits bei der Immobilisierung eines Regulators des Komplementsystems auf bestimmten Oberflächen von Biomaterialien eingesetzt werden konnte (siehe Andersson et al. "Binding of a model regulator of complement activation (RCA) to a biomaterial surface: surface-bound factor H inhibits complement activation", Biomaterials 22: 2435-2443, 2001). Mit der Verwendung dieses Linkers war die biologische Aktivität des Regulators nicht beeinträchtigt. PEG-BlockCopolymere, die sich ebenfalls als geeignete Linker erwiesen haben, sind bspw. zusammenfassend dargestellt in Tirelli et al. "Poly(ethylene glycol)block copolymers", Biotechnol. 90(1): 3-15, 2002.

Ferner können die Aptamere prinzipiell wie alle Nukleotide (bspw. nach Kopplung mit Amino- oder Biotingruppen am 3'- oder 5'-Ende) über geeignete Linkermoleküle oder Spacer an der Oberfläche der Implantate angebracht werden. Verfahren zur Immobilisierung von Oligonukleotiden sind bspw. beschrieben in "Immobilisierung von Oligonucelotiden an aminofunktionalisierte Silizium-Wafer" (U. Haker, Chem. Diss., Hamburg, 2000), wobei hier u.a. 1,4-Phenylendiisothiocyanat eingesetzt wird. In der Dissertation "Miniaturisierte Affinitätsanalytik - Ortsaufgelöste Oberflächenmodifikationen, Assays und Detektion" (I. Stemmler, Chem. Diss., Tübingen, 1999) und in der Veröffentlichungen von Hermanson et al., "Immobilized affinity ligand techniques" (Academic Press, San Diego, 1992) und "Bioconjugate Techniques" (academic Press, San Diego, 1996) sind weitere wichtige kovalente Verfahren zur Oberflächenmodifikation dargestellt. So können bspw. als funktioneller Anker SiO₂, TiO₂, - COOH, HfO₂, -Au, -Ag, N-Hydroxysuccinimid, -NH2, Epoxid, Maleinimid, Säurehydrazid, Hydrazid, Azid, Diazirin, Benzophenon, u.a., mit verschiedenen Reaktionspartnern bei Kupplungen eingesetzt werden.

Eine weitere Methode zur Immobilisierung von Oligonukleotiden auf Oberflächen stellt das Photolinking dar. Dabei wird das NH₂-gekoppelte Oligonukleotid (Aptamer) zunächst mit einem sogenannten Photolinker-Molekül versehen (z.B. Anthraquinone), welches später unter UV-Aktivierung photochemische Reaktionen mit einer Kunststoffoberfläche eingehen kann und das Oligonukleotid somit kovalent an die Oberfläche bindet. Kits und Substanzen zur Durchführung dieses Verfahrens sind bspw. unter der Bezeichnung AQ-Link™ und DNA Immobilizer™ von der Firma Exiqon (Vedbaek, Dänemark) kommerziell erhältlich.

Die Erfinder konnten in eigenen Versuchen zeigen, dass über bestimmte ausgewählte Aptamere, insbesondere über diejenigen, die ein Nukleinsäuremolekül mit der Nukleotidsequenz SEQ ID-Nr. 1 bis SEQ ID-Nr. 17 aufweisen, endotheliale Vorläuferzellen gebunden werden. Auf diese Weise können bspw. aptamerbeschichtete Oberflächen von Stents Endothelzellen binden, wodurch diese optimal an das die Gefäße auskleidende Gewebe angepasst werden können.

Durch diese Zell- und/oder Protein-Bindung an die Aptamerbeschichtete Oberfläche wird vorteilhaft eine autologe Oberflächenstruktur geschaffen, wodurch bspw. bei Implantaten ein "host versus graft response" und damit Implantations-Folgekosten vermieden werden, die oftmals bei solchen Reaktionen entstehen.

In einer weiteren Ausführungsform kann das erfindungsgemäße Implantat zusätzlich mit Wachstumsfaktoren beschichtet sein.

Diese Ausführungsform hat den Vorteil, dass endotheliale Vorläuferzellen (endothelial progenitor cells, EPC), über Aptamere an die Oberflächen gebunden werden und mittels spezifischer Wachstumsfaktoren, die als Induktoren wirken, zu vollwertigem Endothel differenziert werden. Diese Wachstumsfaktoren können dabei bspw. ebenfalls über Aptamere an der Oberfläche der Vorrichtung immobilisiert werden (Co-Immobilisation). Dabei ist bevorzugt, wenn die Wachstumsfaktoren ausgewählt sind aus der Gruppe umfassend den "Platelet derived Growth Factor" (PDGF), den "Vascular Endothelial Growth Factor" (VEGF), den "Colony Stimulating Factor" (CSF), den "Epidermal Growth Factor" (EGF), den "Nerve Growth Factor" (NGF), den Fibroblast Growth Factor (FGF), und/oder Wachstumsfaktoren aus der Reihe der TGF-Superfamilie. Wachstumsfaktoren aus der Reihe der TGF(Transforming Growth Factor)-Superfamilie sind bspw. BMP's (Bone Morphogenetic Proteins) wie BMP-2 und BMP-7.

So können bspw. derart vorbehandelte Gefäßprothesen sofort nach der Implantation aus dem durch sie zirkulierenden Blut EPC's an die Oberfläche binden und das Prothesenmaterial innerhalb kürzester Zeit endothelialisieren.

Weiterhin ist in einer Ausführungsform bevorzugt, wenn die Aptamere eine DNAzym-Aktivität aufweisen.

Dies hat den Vorteil, dass eine zumindest teilweise enzymatisch wirksame Beschichtung bereitgestellt wird. Werden spezifisch Aptamere mit DNAzym-Aktivität eingesetzt, wird dadurch diejenige mRNA abgebaut, welche durch die DNAzyme erkannt wird.

Dies kann bspw. bei der Regulation der Egr-1-Protein-Produktion von Vorteil sein. Das Egr-1-Protein ist ein Protein, das beim Wachstum von glatter Muskulatur benötigt wird. Durch den Einsatz von derartig beschichteten Gefäßprothesen wird bspw. das Zuwachsen von Gefäßen verhindert. Ferner können mit solchen enzymatisch aktiven Aptameren bspw. Blutgerinnungskaskaden reguliert werden.

Bei einer weiteren Ausführungsform ist bevorzugt, wenn als Oberfläche ein Material eingesetzt wird, das aus der Gruppe umfassend Polytetrafluorethylen, Polystyrol, Polyurethan, Polyester, Polylactid, Polyglykolsäure, Polysulfon, Polypropylen, Polyethylen, Polycarbonat, Polyvinylchlorid, Polyvinyldifluorid, Polymethylmethacrylat, Polyethylenterephthalat, ePTFT, Texin (Polyether-Polyurethan) oder Copolymere davon, Nylon, silanisiertes Glas, Keramik oder Metall, insbesondere Titan, oder Mischungen davon ausgewählt ist.

Ferner können als Material auch Nanomaterialien eingesetzt werden und/oder Nanomaterialien, die aus DNA-Bausteinen bestehen und die einen gewissen Prozentsatz an Aptameren enthalten.

Derartige Materialien haben sich in den Fachgebieten, die sich bspw. mit dem Tissue-Engineering oder insgesamt mit der Gefäßchirurgie beschäftigen, bewährt, und werden in verschiedenen Ausführungsformen eingesetzt.

Die Form der Oberfläche kann dabei beliebig ausgewählt sein.

Als Implantate kommen insbesondere Kunstherzen, Herzklappen, Gefäßprothesen, künstlichen Organen, Stents, künstliche Hüften, Knochen, Sehnen, Bänder, Gelenke, Knorpel, dentale Implantate, künstliche Cornea, Haut, Darm, intraoculare Linsen, azellularisierte Organe, Gefäßimplantate etc., bei denen nur noch das ursprüngliche Stützgerüst vorhanden ist, und viele andere in Frage. Bei solchen Oberflächen besteht das Bedürfnis, eine gezielte Zelladhäsion zu bewirken.

Die erfindungsgemäß beschichteten Implantate werden entweder *in vivo*, also *in situ* mit Zellen beschichtet, d.h. direkt in dem Patienten, in welchem sich dann auf dem Implantat das autologe Gewebe bildet, oder aber *ex vivo* oder *in vitro*.

Ferner können die erfindungsgemäß beschichteten Implantate als Bioreaktoren zur Isolierung von bestimmten Zelltypen und anschließender Vermehrung zur Produktion von bestimmten Substanzen oder als Organersatz (Leber, Pankreas, etc.) eingesetzt werden.

Die Erfindung betrifft ferner Nukleinsäuremoleküle mit einer der Nukleotidsequenzen 1 bis 17 aus dem beiliegenden Sequenzprotokoll.

Die Erfinder haben erkannt, dass es mit den Nukleinsäuremolekülen möglich ist, gezielt endotheliale Vorläuferzellen zu immobilisieren.

Solch modifizierte Nukleinsäuremoleküle bzw. Aptamere haben gegenüber den in der Diagnostik üblicherweise verwendeten monoklonalen Antikörpern eine Vielzahl von Vorteilen. Aufgrund der sequenzbedingten Ausbildung von Sekundärstrukturen ist das Repertoire an potentiell bindenden Liganden wesentlich größer als das verfügbare Immunrepertoire zur Herstellung von monoklonalen Antikörpern.

Ferner sind Aptamere wesentlich schneller und kostengünstiger bereitzustellen. Innerhalb von drei bis vier Wochen lassen sich Millionen potentieller Liganden untersuchen.

Im Rahmen eines diagnostischen Verfahrens eignen sich als Diagnostikum insbesondere fluoreszierende Verbindungen, z.B. Fluorescinisothiocyanat (FITC), Biotin, Dioxygenin und deren Abkömmlinge, enzymatische Marker, Infrarot-Marker und Gelatbildner.

Die Erfindung betrifft ferner die Verwendung der vorstehenden Nukleinsäuremoleküle für die Beschichtung von Oberflächen den Vorgenannten Implantate.

Die Erfinder haben erkannt, dass der direkte Einsatz der beanspruchten Nukleinsäuremoleküle als sozusagen "Fängermolekül" möglich ist, was eine *in situ*-Immobilisierung der biologischen Zielstruktur innerhalb von Geweben und/oder Flüssigkeiten erlaubt.

Die Erfindung betrifft weiterhin ein Verfahren zur Beschichtung von Implantaten mit zumindest einer mit Geweben und/oder Flüssigkeiten in Kontakt kommenden Oberfläche, welche zumindest teilweise mit Substanzen beschichtet ist, die die Adhäsion von endothelialen Vorläuferzellen vermitteln, mit den folgenden Schritten:
a) Bereitstellen von Aptameren, die die Adhäsion von endothelialen Vorläuferzellen vermitteln,
b) Binden der Aptamere aus Schritt a) an die Oberfläche einer Vorrichtung.

Es ist dabei bevorzugt, wenn als Aptamere Nukleinsäuremoleküle verwendet werden, die zumindest eine der Nukleotidsequenzen SEQ ID-Nr. 1 bis SEQ ID-Nr. 17 aus dem beiliegenden Sequenzprotokoll enthalten.

Die mit diesem Verfahren hergestellten Implantate können direkt als Implantat eingesetzt werden.

Weitere Vorteile ergeben sich aus den Figuren und dem nachstehenden Beispiel.

Ausführungsbeispiele der Erfindung sind in der Zeichnung dargestellt und werden in der nachfolgenden Beschreibung näher erläutert. Es zeigt:

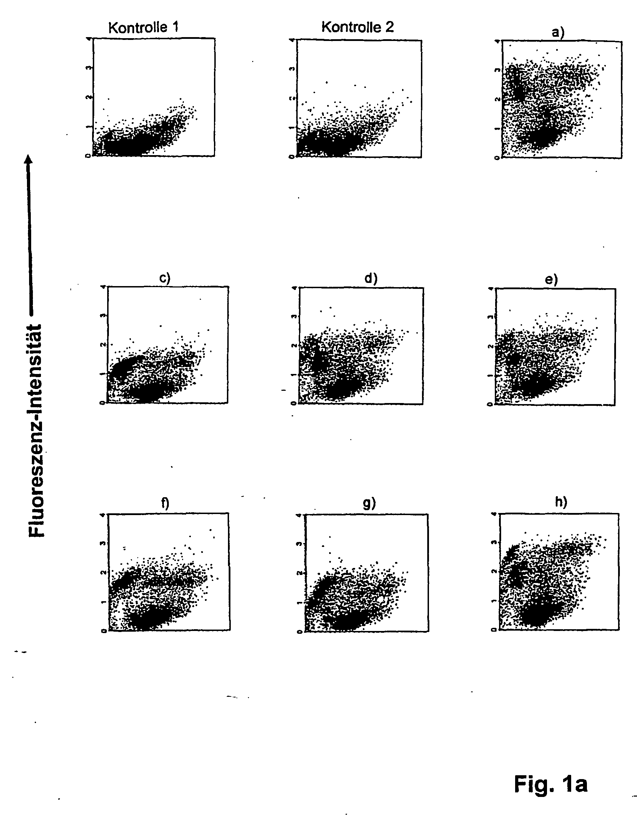

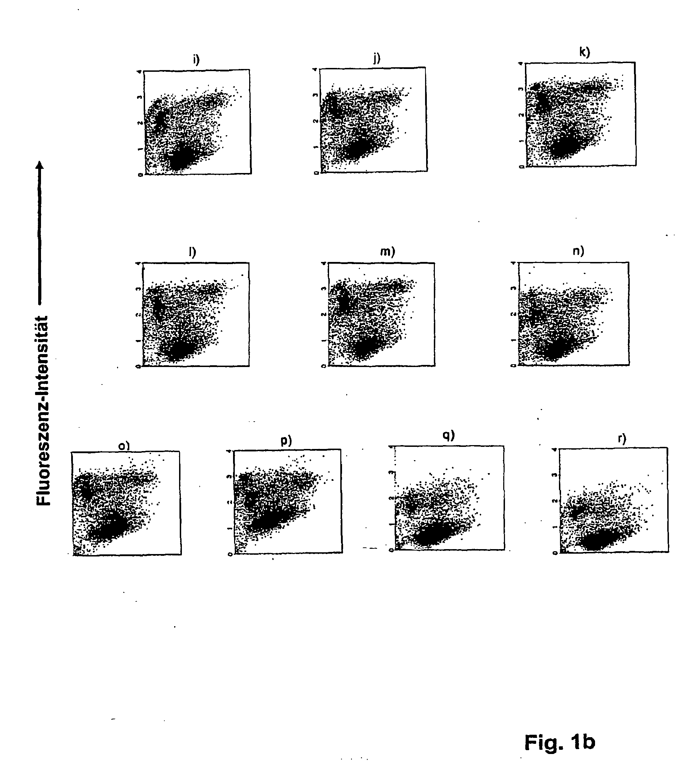

Fig. 1a+b die Charakterisierung der beanspruchten Nukleinsäuremoleküle mittels Durchflusszytometrie.

### Beispiel

### Selektion von Aptameren gegen EPC (endotheliale Vorläuferzellen)

EPC's wurden aus Knochenmark von Lewis-Ratten in kommerziell erhältlichen Selektions- und Propagationsmedien kultiviert.

Aus einer Bibliothek (synthetische Oligonukleotide, MWG Biotech, Deutschland) von DNA-Oligonukleotiden, von denen bekannt ist, dass sie an interzelluläre Regulations-(Transkriptions-) Faktoren binden, wurden Oligonukleotide (Aptamere) selektiert, die an EPC's binden. Dabei wurde unter Verwendung von EPC's das gleiche Verfahren angewandt wie bereits in der DE 100 19 154 und in der Veröffentlichung "Systematic evolution of a DNA aptamer binding to rat brain tumor microvessels: selective targeting of endothelial regulatory protein pigpen" (Blank et al., J. Biol. Chem. 2001; 276(19):16464-8) beschrieben.

In der folgenden Tabelle 1 sind die Sequenzen und Bezeichnungen der identifizierten, an EPC bindenden Oligonukleotide gezeigt:

| FACS-Nr. | Oligonukleotide | Sequenzen | Ergebnisse |
|---|---|---|---|
| Kontrolle 1 | Zellen | | neg. |
| Kontrolle 2 | SEL III 11-1 | ctg ttg gac att caa aag ac | neg. |
| a) | 4 cpg FITC | tcg tcg ttt tgt cgt ttt gtc gt | pos. |
| c) | Trirep 3 | ccg ccg ccg ccg ccg ccg ccg | pos. |
| d) | Trirep 5 | gcg gcg gcg gcg gcg gcg gcg | pos. |
| e) | Trirep 6 | cgg cgg cgg cgg cgg cgg cgg | pos. |
| f) | Trirep 8 | ttc ttc ttc ttc ttc ttc ttc | pos. |
| g) | Trirep 10 | tta ggt tag gtt agg tta ggt tag g | pos. |
| h) | Cpgoligo 1 | gct aga cgt tag cgt | pos. |
| i) | Cpgoligo 1 control | gct aga gct tag gct | pos. |
| j) | Cpgoligo 2 | gat tgc ctg acg tca gag ag | pos. |
| k) | Cpgoligo 3 | ttc atg acg ttc ctg atc gt | pos. |
| I) | Cpgoligo 3 control 1 | tcc atg act ttc ctc agg tt | pos. |
| m) | Cpgoligo 3 control 2 | tcc atg agc ttc ctg atg ct | pos. |
| n) | Cpgoligo 4 control | tgc tgc ttt tgt gct ttt gtg ctt | pos. |
| o) | dnazegr 1 | ccg cgg cca ggc tag cta caa cga cct gga cga t | pos. |
| p) | aptzymegr 1 | att gtg gtt ggt agt ata cat ttt tcc gcg gcc agg cta gct aca acg acc tgg acg at | pos. |
| q) | Soxs-2-78-113 | ctt taa tgc ggg gta att tct tttt cca taa tcg c | pos. |
| r) | Cysk-2-66-106 | tta ttt ccc tte tgt ata tag ata tgc taa atc ctt act t | pos. |

Die Oligonukleotide wurden FITC- (Fluoresceinisothiocyanat) markiert und deren Bindung an EPC's mittels Durchflusszytometrie (FACS) nachgewiesen. Die Ergebnisse dieser Analysen sind in den Figuren 1a und 1b gezeigt und in Tabelle 1 zusammengefasst. In der Tabelle 1 bedeutet "pos." die Bindung der Zellen (EPC's) an die FITC-markierten Aptamere. Als Kontrolle wurde das Oligonukleotid SEL III 11-1 eingesetzt, das nachgewiesenermaßen nicht an EPC bindet. Die Oligonukleotide a) bis r) aus der- Tabelle 1 wurden zytometrisch getestet, die Ergebnisse dieser Analysen sind in den Plots a) bis r) der Fig. 1a und b wiedergegeben, die jeweils den eingesetzten Oligonukleotiden a) bis r) entsprechen.

Wie in den Plots a) bis r) der Figuren 1a und 1b zu erkennen ist, finden sich mit den identifizierten Oligonukleotiden durchweg positive Bindungsreaktionen. Die Oligonukleotide o) und p) wurden als DNAzyme identifiziert.

Die im Sequenzprotokoll aufgeführten Sequenzen 1 bis 17 entsprechen den in der Tabelle 1 angegebenen Oligonukleotiden a) bis r).

Für die Messung von Immobilisierungsvorgängen wird eine Durchflusszelle verwendet, die sich unter einem Fluoreszenzmikroskop befindet. In der Durchflusszelle befindet sich Aptamerbeschichtetes Trägermaterial- (Linker: Photolinker, AQ Photochemistry, Exiqon, Dänemark). Die Zelle wird mit Zellsuspensionen, Proteinlösungen, Plasma, Blut oder anderen relevanten biologischen Lösungen perfundiert. Das Target (Protein, Zelle, o.a.) ist dabei Floureszenz-markiert. Die Geschwindigkeit der Anlagerung der Targets (Herausfischen oder "Capture") kann bspw. mit einer Videokamera dokumentiert werden.

Anstelle einer Durchflusszelle kann auch eine handelsübliche ELISA-Platte eingesetzt werden, die mit dem Aptamer beschichtet wird. Über einen markierten Antikörper kann nach Standard-ELISA-Techniken das Target quantifiziert werden.

## Patentansprüche

1. Implantat mit zumindest einer mit Geweben und/oder Flüssigkeiten des menschlichen oder tierischen Körpers in Kontakt kommenden Oberfläche, welche zumindest teilweise mit Substanzen beschichtet ist, die die Adhasion von endothelialen Vorläuferzellen (EPC) vermitteln, **dadurch gekennzeichnet, dass** die Substanzen Aptamere sind.

2. Implantat nach Anspruch 1, **dadurch gekennzeichnet, dass** die Aptamere Nukleinsäuremoleküle sind; die zumindest eine der Sequenzen SEQ ID-Nr. 1 bis SEQ ID-Nr. 17 aus dem beilegenden Sequenzprotokoll enthalten.

3. Implantat nach Anspruch 1, **dadurch gekennzeichnet, dass** die Aptamere Nukleinsäuremoleküle mit zumindest einer der Nukleotidsequenzen SEQ ID-Nr. 1 bis SEQ ID-Nr. 17 aus dem beiliegenden Sequenzprotokoll sind.

4. Implantat nach einem der Ansprüche 1 bits 3, **dadurch gekennzeichnet, dass** die Aptamere direkt und/oder über ein Linkermolekül an der Oberfläche angebracht sind.

5. Implantat nach einem der Ansprüche 1 bis 4, **dadurch gekennzeichnet, dass** es zusätzlich Wachstumsfaktoren aufweist.

6. Implantat nach Anspruch 5, **dadurch gekennzeichnet, dass** die Wachstumsfaktoren ausgewählt sind aus der Gruppe enthaltend "Platelet derived Growth Factor" (PDGF), "Vascular Endothelial Growth Factor" (VEGF), "Colony Stimulating Factor" (CSF), "Epidermal Growth Factor" (EGF), "Nerve Growth Factor" (NGF), "Fibroblast Growth Factor (FGF) und/oder' Wachstumsfaktoren aus der "Transforming Growth Factor"(TGF)-Superfamilie.

7. Implantat nach einem der Ansprüche 1 bis 6, **dadurch gekennzeichnet, dass** die Aptamere eine DNAzym-Aktivität aufweisen.

8. Implantat nach einem der Ansprüche 1 bis 7, **dadurch gekennzeichnet, dass** die Oberfläche ein Material aufweist, das aus der Gruppe umfassend Polytetrafluorethylen, Polystyrol, Polyurethan, Polyester, Polylactid, Polygykolsäure, Polysulfon, Polypropylen, Polyethylen, Polycarbonat, Polyvinylchlorid, Polyvinyldifluorid, Polymethylmethacrylat, Polyethylenterephthalat, ePTFT, Texin (Polyether-Polyurethan) oder Copolymere davon, Nylon, silanisiertes Glas, Keramik, Metalle, insbesondere Titan, oder Mischungen davon, ausgewählt ist.

9. Verwendung eines Nukleinsäuremoleküls mit einer der Nukleotidsequenzen SEQ ID-Nr. 1 bis SEQ ID-Nr. 17 aus dem beiliegenden Sequenzprotokoll zur Beschichtung von Oberflächen eines Implantates nach einem der Ansprüche 1 bis 11, um die Adhäsion von endothelialen Vorläuferzellen ((EPC) )zu vermitteln

10. Verfahren zur Herstellung von Implantaten mit zumindest einer mit Geweben und/oder Flüssigkeiten des menschlichen oder tierischen Körpers in Kontakt kommenden Oberfläche, welche zumindest teilsweise mit Substanzen beschichtet ist, die die Adhäsion von endothelialen Vorläuferzellen (EPC) vermitteln, mit den folgenden Schritten:
a) Bereitstellen von Aptameren, die die Adhäsion von endothelialen Vorläuferzellen (EPC) vermitteln,
b) Binden der Aptamere aus Schritt a) an die Oberfläche eines Implantates.

11. Verfahren nach Anspruch 10, **dadurch gekennzeichnet, dass** als Aptamere Nukleinsäuremoleküle verwendet werden, die zumindest eine der Nukleotidsequenzen SEQ ID-Nr. 1 bis SEQ ID-Nr. 17 aus dem beiliegenden Sequenzprotokoll enthalten.

12. Verfahren nach Anspruch 11, **dadurch gekennzeichnet, dass** als Aptamere Nukleinsäuremoleküle mit zumindest einer der Nukleotidsequenzen SEQ ID-Nr. 1 bis SEQ ID-Nr. 17 aus dem beiliegenden Sequenzprotokoll verwendet werden.

## Claims

1. An implant comprising at least one surface which comes into contact with tissues and/or fluids of the human or animal body, and which is at least partially coated with substances which mediate the adhesion of endothelial progenitor cells (EPC), **characterized in that** the substances are aptamers.

2. The implant of claim 1, **characterized in that** the aptamers are nucleic add molecules which comprise at least one of the sequences of SEQ ID No. 1 to SEQ ID No. 17 from the enclosed sequence listing.

3. The implant of claim 1, **characterized in that** the aptamers are nucleic acid molecules having at least one of the nucleotide sequences of SEQ ID No. 1 to SEQ ID No. 17 from the enclosed sequence listing.

4. The implant of any of claims 1 to 3, **characterized in that** the aptamers are attached to the surface directly and/or by way of a linker molecule.

5. The implant of any of claims 1 to 4, **characterized in that** it additionally comprises growth factors.

6. The implant of claim 5, **characterized in that** the growth factors are selected from the group consisting of: platelet-derived growth factor (PDGF), vascular endothelial growth factor (VEGF), colony-stimulating factor (CSF), epidermal growth factor (EGF), nerve growth factor (NGF), fibroblast growth factor (FGF), and growth factors from the transforming growth factor (TGF) super-family.

7. The implant of any of claims 1 to 6, **characterized in that** the aptamers exhibit a DNAzyme activity.

8. The implant of any of claims 1 to 7, **characterized in that** the surface comprises a material which is selected from the group consisting of: polytetrafluoroethylene, polystyrene, polyurethane, polyester, polylactide, polyglycolic acid, polysulfone, polypropylene, polyethylene, polycarbonate, polyvinyl chloride, polyvinyl difluoride, polymethyl methacrylate, polyethylene terephthalate, ePTFT, texin (polyether-polyurethane) and copolymers thereof, nylon, silanized glass, ceramic and metals, in particular titanium, and mixtures thereof.

9. Use of a nucleic acid molecule comprising a nucleotide sequence of SEQ ID No. 1 to SEQ ID No. 17 from the enclosed sequence listing for coating surfaces of an implant of any of claims 1 to 8, to mediate the adhesion of endothelial progenitor cells (EPC).

10. A method for producing implants comprising at least one surface which comes into contact with tissues and/or fluids of the human or animal body, and which is at least partially coated with substances which mediate the adhesion of endothelial progenitor cells (EPC), comprising the following steps:
a) providing aptamers which mediate the adhesion of endothelial progenitor cells (EPC),
b) binding the aptamers from step a) to the surface of a device.

11. The method of claim 10, **characterized in that** the aptamers employed are nucleic acid molecules which comprise at least one of the nucleotide sequences of SEQ ID No. 1 to SEQ ID No. 17 from the enclosed sequence listing.

12. The method of claim 11, **characterized in that** the aptamers employed are nucleic acid molecules having at least one of the nucleotide sequences of SEQ ID No. 1 to SEQ ID No. 17 from the enclosed sequence listing.

## Revendications

1. Implant avec au moins une surface venant en contact avec des tissus et/ou des liquides du corps humain ou animal, laquelle est revêtue au moins en partie par des substances qui procurent l'adhésion de cellules endothéliales précurseuses (E.P.C.), **caractérisé en ce que** les substances sont des aptamères.

2. Implant selon la revendication 1, **caractérisé en ce que** les aptamères sont des molécules d'acide nucléique qui contiennent au moins une des séquences SEQ ID NO 1 à SEQ ID NO 17 du listage de séquences.

3. Implant selon la revendication 1, **caractérisé en ce que** les aptamères sont des molécules d'acide nucléique avec au moins une des séquences SEQ ID NO 1 à SEQ ID NO 17 du listage de séquences.

4. Implant selon l'une quelconque des revendications 1 à 3, **caractérisé en ce que** les aptamères sont appliqués sur la surface directement et/ou par l'intermédiaire d'une molécule d'attache.

5. Implant selon l'une quelconque des revendications 1 à 4, **caractérisé en ce qu'**il comporte en plus des facteurs de croissance.

6. Implant selon la revendication 5, **caractérisé en ce que** les facteurs de croissance sont choisis parmi le groupe contenant le « Platelet derived Growth Factor » (PDGF), le « Vascular Endothelial Growth Factor » (VEGF), le « Colony Stimulating Factor » (CSF), 1'« Epidermal Growth Factor » (EGF), le « Nerve Growth Factor » (NGF), le « Fibroplast Growth Factor » (FGF) et/ou des facteurs de croissance provenant de la superfamille du « Transforming Growth Factor » (TGF).

7. Implant selon l'une quelconque des revendications 1 à 6, **caractérisé en ce que** les aptamères présentent une activité ADN-enzymatique.

8. Implant selon l'une quelconque des revendications 1 à 7, **caractérisé en ce que** la surface comporte un matériau qui est choisi parmi le groupe comprenant le polytétrafluoréthylène, le polystyrène, le polyuréthane, le polyester, l'acide polylactique, l'acide polyglycolique, la polysulfone, le polypropylène, le polyéthylène, le polycarbonate, le polychlorure de vinyle, le difluorure de polyvinyle, le polyméthylméthacrylate, le poly(téréphtalate d'éthylène), le ePTFT, le Texin (polyéther-polyuréthane) ou des copolymères de ces derniers, le nylon, le verre siliconé, la céramique, les métaux, en particulier le titane, ou des mélanges de ceux-ci.

9. Utilisation d'une molécule d'acide nucléique avec une des séquences de nucléotides SEQ ID NO 1 à SEQ ID NO 17 du listage de séquences pour le revêtement des surfaces d'un implant selon l'une quelconque des revendications 1 à 8, pour réaliser l'adhésion de cellules endothéliales précurseuses (E.P.C.).

10. Procédé pour la fabrication d'implants avec au moins une surface venant en contact avec des tissus et/ou des liquides du corps humain ou animal, laquelle est revêtue au moins en partie par des substances qui procurent l'adhésion de cellules endothéliales précurseuses (E.P.C.), avec les étapes suivantes :
a) mise à disposition d'aptamères qui procurent l'adhésion de cellules endothéliales précurseuses (E.P.C.),
b) fixation des aptamères de l'étape a) à la surface d'un implant.

11. Procédé selon la revendication 10, **caractérisé en ce que** des molécules d'acide nucléique qui contiennent au moins une des séquences de nucléotides SEQ ID NO 1 à SEQ ID NO 17 du listage de séquences sont utilisées comme aptamères.

12. Procédé selon la revendication 11, **caractérisé en ce que** des molécules d'acide nucléique avec au moins une des séquences de nucléotides SEQ ID NO 1 à SEQ ID NO 17 du listage de séquences sont utilisées comme aptamères.
